# EUROPEAN PATENT APPLICATION

(11) **EP 3 797 773 A1**
(43) Date of publication of application: **31.03.2021**
(21) Application number: 19020505.4
(22) Date of filing: 03.09.2019
(51) Int. Cl.: A61K 31/4704, A61P 35/00

(54) **REBAMIPIDE FOR USE IN PROPHYLAXIS AND TREATMENT OF CANCER**

(71) Applicant: SQUARE POWER LTD, London EC2A 3DQ (GB)
(72) Inventor:

(57) **Abstract**

The present invention relates to rebamipide for use in a method of prophylaxis and/or treatment of cancer in a person suffering from increased intestinal permeability or in a person which is at risk of increased intestinal permeability, e.g. due to family anamnesis or due to exposure to conditions or substances inducing increased intestinal permeability.

## Description

### Field of the Invention

The present invention relates to rebamipide for use in a method of prophylaxis and/or treatment of cancer in a person suffering from or being at risk of increased intestinal permeability.

### Background Art

Cancer is one of the leading causes of death worldwide responsible for 25% of deaths in Europe. It's a disease caused by genetic changes, that can be either sporadic or hereditary, leading to uncontrolled cell growth and tumor formation. Most cancers are related to environmental, lifestyle, or behavioral exposures. Common environmental factors that contribute to cancer include various carcinogens, such as high energy radiation, including UV light, x-ray and gamma radiation, tobacco smoke, alcohol beverages, some microorganisms and parasites, and various chemical compounds.

Diet is thought to play a substantial role in cancer etiology with a growing evidence that some dietary habits increase the risk of cancer. These include diets with high glycemic load, rich in fat and with low antioxidant content to name just a few. Moreover, certain types of food contain substances known to promote cancer development. These can be either natural products or anthropogenic chemicals that are either present as a contamination or are intentionally added to food, serving as preservatives, colorants, flavorants, etc.

Chronic inflammation, even of a low-grade nature, represents another important risk factor in cancer development. The inflammatory microenvironment, consisting of a variety of immune cells, epithelial cells, stromal cells, cytokines, and chemokines, has many similarities to the microenvironment of cancers, suggesting similar inflammatory mediators and mechanisms that promote both chronic inflammation and cancer development. These mediators, produced by inflammatory cells, include tumor necrosis factor alpha (TNF-α), ILs-1, 6, 12, 13, 17, 22, and 23. The exact molecular mechanisms that underlie the transition from inflammation to cancer are not fully understood. The current body of evidence suggests that multiple factors play roles in cancer development: immune response, activation of oncogenes, inhibition of tumor suppressors, modifications to normal microRNA expression patterns, alterations to the epigenetic landscape, as well as commensal microbiota, and the corresponding inflammatory response of the epithelial cells.

Although a number of resources have been utilized for the development of cancer therapeutics, no effective cure has been found so far. Therefore, there is a high unmet medical need for new medicines that could be used in the treatment or prevention of cancer.

Rebamipide, which is chemically 2-[(4-chlorobenzoyl)amino]-3-(2-oxo-1H-quinolin-4-yl)propanoic acid) is used for the treatment of acute and/or chronic gastritis and gastroduodenal ulcers. Its mechanism of action relates to mucosal defense, scavenging free radicals, and temporarily activating genes encoding cyclooxygenase-2.

### Disclosure of the Invention

In the framework of the present invention, it was found that rebamipide is capable of preventing and/or treating cancers associated with increased intestinal permeability. Under normal physiological conditions the intestinal wall works as a barrier effective at absorbing nutrients, but preventing passage of harmful molecules and microorganisms from inside the bowel into the body proper. However, if the barrier is damaged for whatever reason, it doesn't work properly allowing substances that would normally stay in the intestinal lumen to pass into the organism. Since human diet contains not just nutrients but also substances bearing carcinogenic potential, such as those occurring in burned food, processed or red meat, increased intestinal permeability represents a serious risk factor in cancer development. Moreover, passage of undesirable foreign substances into the intestinal mucosa and the body proper, results in activation of the immune system and secretion of inflammatory mediators leading to chronic intestinal inflammation, often subclinical in nature, that further supports both intestinal permeability and cancer development.

Rebamipide presumed mechanism of action is likely based on its ability to induce mucine production in the intestine, to suppress inflammation and to restore the function of the tight junctions of epithelial cells, thus reducing the permeability of the intestinal wall. This leads to recovery of the intestine, resulting in restoration of the immunological barrier in the intestine that prevents further tissue damage by toxins or pathogens present in the gastrointestinal tract, thus preventing chronic inflammation and subsequent development of malignancies either in the gastrointestinal tract or elsewhere in the body.

The present invention thus provides rebamipide for use in a method of prophylaxis and/or treatment of a cancer in a person suffering from increased intestinal permeability or in a person which is at risk of increased intestinal permeability, e.g. due to family anamnesis or due to exposure to conditions or substances inducing increased intestinal permeability. In a preferred embodiment rebamipide is for use in a method of prophylaxis of a cancer, especially if related to diet or intestinal inflammation.

"Rebamipide", as used herein, shall include all forms of this active ingredient, such as anhydrous form, hydrated or solvated form (e.g. hemihydrate form), crystalline forms; and pharmaceutically acceptable salts thereof.

"Cancer" as used herein is a group of diseases involving abnormal cell growth with the potential to invade or spread to other parts of the body. This invention relates specifically to cancers associated with increased intestinal permeability, a condition that enables passage of carcinogens present in the gastrointestinal tract into the body proper, thus contributing to development of malignancies. These carcinogens may trigger cancerous growth directly in the bowel and/or surrounding tissues but also in more distant organs and tissues. The underlying mechanism for this phenomena resides in the ability of carcinogens to spread via bloodstream even to remote parts of the body.

"Carcinogens" are substances and their mixtures that after entering the body are able to trigger processes leading to cancer development. This may be due to their ability to damage the genome or to disrupt cellular metabolic processes. They form a heterogeneous group classified by the International Agency for Research on Cancer (IARC) into several groups based on the strength of evidence for carcinogenicity. While some substances may be carcinogenic by themselves, others may become carcinogenic only after being metabolized either in the intestine or elsewhere in the body. This invention relates preferably to those carcinogens that under normal physiological conditions are unable to cross the gut wall or cross it at a relatively low rate, but their passage into the body proper is substantially increased if a person suffers from the increased intestinal permeability.

In one embodiment, the invention provides rebamipide for use in a method of prophylaxis and/or treatment of a diet-related cancer. Diet-related cancers are malignancies caused by carcinogens present in food, where they occur either naturally or as a contamination, or are intentionally added as food additives. Persons suffering from or being at risk of increased intestinal permeability are more sensitive to various food-borne carcinogens, such as those occurring in burned food, processed or red meat.

Processed meat refers to meat that has been transformed through salting, curing, fermentation, smoking, or other processes to enhance flavor or improve preservation. The IARC classified it as a group 1 carcinogen, which means it is carcinogenic to humans. Red meat refers to all mammalian muscle meat, including beef, veal, pork, lamb, mutton, horse, and goat and was classified as group 2A carcinogen, which means probably carcinogenic to humans. Current research shows that these foods contain both added and naturally occurring carcinogens including acrylamide, polycyclic aromatic hydrocarbons (PAHs), such as benzo[a]pyrene, benzo[a]anthracene, benzo[b]fluoranthene and chrysene; heterocyclic aromatic amines (HCAs), such as 2-amino-3-methylimidazo[4,5-f]quinoline (IQ), 2-Amino-3,4-dimethylimidazo [4,5-f]quinoline (MeIQ), 2-amino-3,8-dimethylimidazo[4,5-f]quinoxaline (MeIQx), 2-amino-1-methyl-6-phenylimiazo[4,5-b]pyridine (PhIP); and N-nitrosamines, such as N-methyl-N'-nitro-N-nitrosoguanidine (MNNG), N-nitrosodi-N-butylamine, N-nitrosodiethylamine (DEN), N-nitrosodimethylamine (NDMA), N-nitrosopiperidine, N-nitrosopyrrolidine, and N-nitrososarcosine.

Other carcinogens entering gastrointestinal tract and causing diet-related cancers originate in microbial contamination of food, which is responsible for production of various and often carcinogenic toxins, such as mycotoxins like aflatoxins, ochratoxins and fumonisins. Furthermore, certain microorganisms present in the gastrointestinal tract may produce and release various carcinogenic compounds, such as nitrosamines, *in situ.*

Food may also be contaminated by many other compounds that can directly or indirectly lead to cancer development including pesticide residues, aromatic compounds, such as benzene, polychlorinated and polybrominated dibenzodioxins, dibenzofurans and biphenyls, such as 2,3,7,8-tetrachlorodibenzo-p-dioxin, heavy metals, such as lead, arsenic, cadmium and their compounds, compounds released from packaging materials, such as formaldehyde, vinyl chloride or bisphenol A.

Cancers to be treated in line with the present invention include both intestinal and non-intestinal malignancies. Intestinal malignancies form a group of cancers affecting small intestine, colon, rectum and anus and comprise adenocarcinomas, squamous cell tumors, carcinoid tumors, intestinal lymphomas, gastrointestinal stromal tumors, leiomyosarcomas, and melanomas. However, they can also affect neighboring tissues, such as pancreas, liver and gallbladder. In a preferred embodiment intestinal cancer is selected from small intestinal cancer, colon cancer, colorectal carcinoma, pancreatic cancer, hepatic cancer, and cholangiocarcinoma. Non-intestinal malignancies include oral cavity cancer, pharyngeal cancer, salivary gland cancers, esophageal cancer, gastric cancer, breast cancer, prostate cancer, ovarian cancer, bladder cancer, lung cancer, skin cancer, blood vessels cancer, hematopoietic cancers, B-cell lymphoma, and thyroid cancer.

"Increased intestinal permeability" is used herein as a term designating little intestinal wall defects, including those caused by subclinical chronic inflammation (low grade inflammation) of the gut wall. These intestinal wall defects may be manifested e.g. by chronic constipation or gastroparesis. Increased intestinal permeability may be diagnosed using specific tests, such as lactulose-mannitol test (LAMA test; e.g., Sequeira I.R. et al. (2014) PLoS One; 9(6):e99256), A-1-AT test, or zonulin test. Typically, increased intestinal permeability is permeability of the intestinal wall to particles having the size of more than 4 Angstroms in radius.

Substances inducing increased intestinal permeability include non-steroidal anti-inflammatory drugs (NSAIDs), such as acetylsalicylic acid, ibuprofen, naproxen, ketoprofen, fenoprofen, flurbiprofen, diclofenac, ketorolac, etodolac, indomethacin, tolmetin, piroxicam, meloxicam and selective COX-2 inhibitors such as celecoxib and etoricoxib; alcohol; nicotine; food additives; antibiotics; and chemotherapeutics. Thus, simultaneous or sequential coadministration of rebamipide with non-steroidal anti-inflammatory drugs, chemotherapeutics or antibiotics prevents intestinal wall damage and thus prevents or delays the onset of cancers associated with increased intestinal permeability. Prophylactic use of rebamipide may also be useful in persons abusing alcohol, nicotine or other drugs known to damage intestinal wall. The term "abuse" as used herein is meant to include any consumption, which is not necessary for medical reasons and leads to dependency and/or health impairments including low grade inflammation of the gut wall.

Conditions inducing increased intestinal permeability are related to stress, imbalanced diet, allergy, bacterial, viral or parasitic infections and various medical treatments. Such conditions in particular include stress-induced gastritis, alimentary intoxication, disbalance of cholic acids, gastric HCl and pepsin secretion, non-infectious diarrhea, radiation therapy, chemotherapy, infectious or post-infectious impairment of the GIT mucosa, dysmicrobia (e.g. induced by antibiotic treatment).

In one embodiment, the invention provides rebamipide for use in a method of prophylaxis and/or treatment of a cancer in a person suffering from chronic intestinal inflammation, including low grade inflammation of the gut wall. Preferably, the cancer is selected from colorectal cancer, small bowel adenocarcinoma, intestinal lymphoma, anal cancer, and cholangiocarcinoma. Most preferably, the cancer is colorectal cancer.

"Prophylaxis" or "prophylactic use" shall be understood herein as preventing or delaying the onset of the disease including its recurrence in patients with complete or partial remission, e.g. after surgical treatment, chemotherapy, radiation therapy, or immunotherapy. It is also intended to include delaying the progression of the disease. In such a case rebamipide is administered to a patient which is in an initial or early stage of the disease to be treated in order to slow down its progression. The prophylactic effect of rebamipide resides in its ability to prevent passage of carcinogenic substances and other toxins into the body proper, thereby preventing events triggering cancer initiation.

"Treatment" shall be understood herein as a therapy that is able to slow, stop or reverse the disease. It is also meant to cover a reduction or alleviation of symptoms of the disease. "Slowing down" the disease means reducing its progress while not being able to completely stop or reverse it, whereas "stopping" the disease means being able to completely halt its progression.

In the therapeutic indications as described in the present invention, rebamipide may preferably be used in oral pharmaceutical forms such as tablets, capsules, dragees, granules, microgranules (sachets), orodispersible tablets or films, sublingual tablets, crushed tablets, oral solutions, oral suspensions, syrups, mouthwashes or rinses. Alternatively, it can be used in rectal pharmaceutical forms such as suppositories and enemas. Preferably, the oral pharmaceutical forms, such as tablets, capsules, dragees and granules, is a form with enteric release, such as enteric sustained release or enteric controlled release.

The pharmaceutical forms may contain at least one pharmaceutically acceptable excipient selected from fillers, binders, lubricants, glidants, disintegrants/swelling agents, solubilizers, enteric release agents, mucoadhesive components, sustained release agents, preservatives, coatings and colorants. Such excipients are known in the art of pharmaceutical formulation, and the skilled person is capable of selecting suitable excipients for the relevant pharmaceutical forms.

Suitable methods for preparing the pharmaceutical forms and compositions includes the processes of wet granulation or dry granulation of the active ingredient with the auxiliary substances and components, or direct homogenization of the active ingredient with the auxiliary substances and components.

Fillers may preferably be selected from saccharide alcohols (such as mannitol, sorbitol, xylitol), lactose, starch, pregelatinized starch, cellulose, sillicified cellulose, calcium hydrogen phosphate, calcium phosphate, sucrose and calcium sulphate. The fillers may preferably be present in the amount of 5 to 90 wt. %, relative to the total weight of the composition.

Binders may preferably be selected from starch, pregelatinized starch, povidone, copovidone, hydroxypropyl methylcellulose, hydroxypropyl cellulose, ethyl cellulose, cellulose. The binders may preferably be present in the amount of 1 to 20 wt. %, relative to the total weight of the composition.

Lubricants may preferably be selected from magnesium stearate, calcium stearate, stearic acid, polyethylene glycol and sodium stearyl fumarate. The lubricants may preferably be present in the amount up to 5 wt. %, relative to the total weight of the composition.

Glidants may preferably be selected from silica, talc and sodium lauryl sulphate. The glidants may preferably be present in the amount of 0.5 to 10 wt. %, relative to the total weight of the composition.

Swelling and/or disintegrating agents may preferably be selected from crospovidone, copovidone, povidone, croscarmellose, hydroxypropyl methylcellulose, starch, pregelatinized starch, low-substituted hydroxypropyl cellulose, sodium carboxymethyl starch. The swelling / disintegrating agents may preferably be present in the amount of 1 to 50 wt. %, relative to the total weight of the composition.

Solubilizers may preferably be selected from poloxamer, sodium lauryl sulphate, polysorbate, polyoxylated oleic glycerides, glycerol monostearate and cyclodextrins. The solubilizers may preferably be present in the amount up to 30 wt. %, relative to the total weight of the composition.

Enteric release agents may preferably be selected from hydroxypropyl methylcellulose phthalate, poly(methacrylic acid-co-methyl methacrylate), cellulose acetate phthalate, poly(vinyl acetate phthalate), esters of aleuritic acid. The enteric release agents may preferably be present in the amount of 2 to 40 wt. %, relative to the total weight of the composition.

Mucoadhesive components may preferably be selected from propylene glycol alginate, sodium alginate, calcium alginate, potassium alginate, hydroxypropyl methylcellulose, sodium carmellose, polyacrylic acid, polyethylene oxide, povidone and copovidone. The mucoadhesive components may preferably be present in the amount of 5 to 70 wt. %, relative to the total weight of the composition.

Sustained release agents may preferably be selected from cellulose and cellulose ethers such as hydroxypropyl cellulose, hydroxypropyl methylcellulose, carboxymethylcellulose, sodium carboxymethylcellulose, ethyl cellulose, methylcellulose, polyvinyl acetate, alginic acid, propylene glycol alginate, sodium alginate, calcium alginate, potassium alginate, polymethacrylates, guar gum, xanthan gum, carrageenan, castor oil, beeswax, carnauba wax, glycerol palmitostearate, glycerol monostearate, glycerol behenate, stearyl alcohol, polyacrylic acid. The sustained release agents may preferably be present in the amount of 5 to 70 wt. %, relative to the total weight of the composition.

The oral pharmaceutical composition may in some embodiments further contain a pharmaceutically acceptable component capable of forming carbon dioxide upon contact with gastric juices, such component may preferably be selected from carbonates and hydrogen carbonates of alkali metals and alkaline earth metals; and may preferably be present in an amount in the range from 1 to 50 wt. %, relative to the total weight of the composition.

A typical daily dose of rebamipide may range from 1 to 5000 mg for an average human (70 kg weight), more preferably from 50 to 2500 mg, even more preferably from 100 to 1000 mg, and most preferably from 300 to 500 mg. When calculating the daily dose with regard to the body weight of the person, the typical dose ranges from 15 µg/kg/day to 70 mg/kg/day, more preferably from 750 µg/kg/day to 35 mg/kg/day, even more preferably from 1.5 mg/kg/day to 15 mg/kg/day, and most preferably from 4 mg/kg/day to 7 mg/kg/day.

When immediate release formulation of rebamipide is administered, the daily dose is typically divided into several doses, which are administered separately. The daily dose may be divided into two to six separate doses taken twice daily or three times per day or four times per day or five times per day or six times per day. In a preferred embodiment the daily dose is divided into three separate doses administered three times per day, e.g. 100 mg dose administered three times per day. Alternatively, the whole daily dose can be taken at once, especially if it is in the form of a sustained release formulation, e.g. 300 mg dose administered once daily.

### Examples of carrying out the Invention

To study the effect of rebamipide on increased intestinal permeability, colonic inflammation and cancer development, a DSS model combined with PhIP treatment was employed. Male CD-1 mice, divided into three groups, designated A to C, were used in the study. At the beginning, mice in all groups received a single intra gastric administration (200 mg/kg body weight) of PhIP (2-amino-1-methyl-6-phenylimidazo[4,5-b]pyridine), a heterocyclic amine (HCA) present in cooked meat, which possesses carcinogenic potential. Subsequently, all animals were given inflammatory insult using 2% dextran sodium sulfate (DSS) in drinking water for 7 days, followed by 2-4 weeks of recovery period, during which mice were given untreated water. This cycle was repeated up to four times to induce increased intestinal permeability leading to severe colitis with loss of body weight and bloody diarrhea. Subsequently, majority of animals developed multiple colon tumors.

Treatment with DSS (dextran sulfate sodium) induces increased intestinal permeability in mice by binding to medium-chain-length fatty acids present in the colon leading to development of inflammation and colitis. Besides that, the DSS administration leads to a long-term development of dysplasia and even colon cancer in some of the mice treated. PhIP treatment accelerates this process and further increases the incidence of colonic tumors. Another advantage of including PhIP in the protocol is that it allows reduction of the DSS dose in mice, and decreases the mortality from DSS-associated acute colitis.

The carcinogenic process of the DSS/PhIP system has a pathological progression from normal intestinal crypts to the formation of foci harboring aberrant crypts with crypt fission and finally to the emergence of microadenomas. These steps recapitulate the sequence of colorectal cancer formation in humans from increased intestinal permeability and inflammation through dysplasia to carcinoma.

Rebamipide treatment started four weeks prior to carcinogen administration and continued throughout the study. Groups A and B received rebamipide 50 mg/kg/day and 350 mg/kg/day, respectively. Rebamipide was applied within the standard daily feed or by gavage if needed. The treatment lasted for 20 weeks. Mice in control group C did not receive rebamipide or any other protective treatment.

**Table 1: The overall study schedule**

| **Procedure** | **Duration** | **Notes** |
|---|---|---|
| **Induction phase** | 4 weeks | Rebamipide pretreatment |
| | 1 week | 1 intra-gastric (i.g.) dose of PhIP, 200 mg/body weight |
| **Exposure/Treatment phase** | 15 weeks | DSS (1wk) + recovery period (2 wks), 4 cycles leading to increased intestinal permeability and colitis |
| **Close-out** | 1 week | Sacrifice, section, post-mortem analysis |

The observed parameters were as follows:
∘ Number and size of tumors
∘ Histological evaluation of the tumors
∘ Weight loss
∘ Colitis severity assessment
∘ Gut permeability
∘ Cytokines and related markers (IL-6, IL-10, IL-lb, TNFα, iNOS, IL-23p19, MCP 1, MIP 2, CXCL1)
∘ Cell-cell adhesion proteins (Claudin 1, β catenin)
∘ Expression of target genes

The PhIP/DSS treatment caused rapid destruction of the colon mucosa with severe inflammation followed by the development of colonic adenocarcinomas. Interestingly, preliminary results obtained from the study indicate a significant difference between control and rebamipide treated groups. Mice in groups A and B recovered faster from the DSS treatment and also showed lower incidence and size of tumors at the end of the study.

This is a surprising observation since rebamipide is known to induce cyclooxygenase-2 (COX-2) expression, which is elevated in approximately 50% of colorectal adenomas and 85% of adenocarcinomas (Eberhart CE et al. Up-regulation of cyclooxygenase 2 gene expression in human colorectal adenomas and adenocarcinomas. Gastroenterology. 1994;107(4):1183-8; Gupta RA and Dubois RN. Colorectal cancer prevention and treatment by inhibition of cyclooxygenase-2. Nat Rev Cancer. 2001;1(1):11-21; Marnett LJ and Dubois RN. COX-2: a target for colon cancer prevention. Annu Rev Pharmacol Toxicol. 2002;42:55-80) and is associated with a worse survival among CRC patients (Ogino S et al. Cyclooxygenase-2 expression is an independent predictor of poor prognosis in colon cancer. Clin Cancer Res. 2008;14(24):8221-7). The aberrant expression of COX-2 that occurs in the majority of colorectal tumors is thought to play a crucial role during colorectal cancer development.

Results obtained from the study show that the administration of rebamipide supports renewal of the intestinal barrier leading to a decrease in permeability of the gastrointestinal mucosa, thereby preventing entry of carcinogenic substances into the organism, suppresses intestinal inflammation, thus preventing or at least delaying the onset of the symptoms and markers corresponding to colon cancer as a representative of diet and inflammation-related cancers in comparison to untreated animals.

## Claims

1. Rebamipide for use in a method of prophylaxis and/or treatment of cancer in a person suffering from increased intestinal permeability or in a person which is at risk of increased intestinal permeability.

2. Rebamipide for use according to claim 1, wherein the cancer is a diet-related cancer.

3. Rebamipide for use according to claim 2, wherein the diet-related cancer is an intestinal malignancy selected from small intestinal cancer, colon cancer, colorectal carcinoma, pancreatic cancer, hepatic cancer, and cholangiocarcinoma.

4. Rebamipide for use according to claim 2, wherein the diet-related cancer is a non-intestinal malignancy selected from oral cavity cancer, pharyngeal cancer, salivary gland cancers, esophageal cancer, gastric cancer, breast cancer, prostate cancer, ovarian cancer, bladder cancer, lung cancer, skin cancer, blood vessels cancer, hematopoietic cancers, B-cell lymphoma, and thyroid cancer.

5. Rebamipide for use according to any one of claims 1 to 4, wherein the person at risk of increased intestinal permeability is a person suffering from stress, imbalanced diet, bacterial, viral or parasitic infection.

6. Rebamipide for use according to any one of claims 1 to 4, wherein the person at risk of increased intestinal permeability is a person exposed to at least one substance selected from non-steroidal anti-inflammatory drugs, alcohol, nicotine, food additives, chemotherapeutics and antibiotics.

7. Rebamipide for use according to claim 6, wherein rebamipide is co-administered simultaneously or sequentially with non-steroidal anti-inflammatory drug, chemotherapeutic or antibiotic.

8. Rebamipide for use according to claim 6, wherein rebamipide is administered to a person abusing a drug, preferably a drug selected from alcohol and nicotine.

9. Rebamipide for use according to any one of claims 1 to 4, wherein the person at risk of increased intestinal permeability is a person suffering from or exposed to at least one condition selected from: stress-induced gastritis, alimentary intoxication, disbalance of cholic acids, gastric HCl and pepsin secretion, non-infectious diarrhea, radiation therapy, chemotherapy, infectious or post-infectious impairment of the GIT mucosa, dysmicrobia.

10. Rebamipide for use according to claim 1 or 2, wherein the person suffering from increased intestinal permeability is a person suffering from chronic intestinal inflammation, including low grade inflammation of the gut wall.

11. Rebamipide for use according to claims 10, wherein the cancer is selected from the group consisting of colorectal cancer, small bowel adenocarcinoma, intestinal lymphoma, anal cancer, and cholangiocarcinoma.

12. Rebamipide for use according to claims 11, wherein the cancer is colorectal cancer.

13. Rebamipide for use according to any one of the preceding claims, wherein the person is in complete or partial remission of cancer after previous treatment.

14. Rebamipide for use according to any one of the preceding claims, wherein the person is in an initial or early stage of cancer.

15. Rebamipide for use according to any one of the preceding claims, wherein rebamipide is used in a method of prophylaxis of cancer.
